# EUROPÄISCHE PATENTSCHRIFT

(11) **EP 1 850 857 B1**
(45) Veröffentlichungstag und Bekanntmachung des Hinweises auf die Patenterteilung: **22.05.2013**
(21) Anmeldenummer: 06706465.9
(22) Anmeldetag: 28.01.2006
(51) Int. Cl.: A61K 31/618, C07C 69/88, C07C 311/17

(54) **ISOLIERUNG VON ATRARSÄURE, SYNTHESE VON ATRARSÄURE-DERIVATEN SOWIE VERWENDUNG DER ATRARSÄURE UND IHRER DERIVATE ZUR BEHANDLUNG DES PROSTATAKARZINOMS.**
ISOLATION OF ATRARIC ACID, SYNTHESIS OF ATRARIC ACID DERIVATIVES, AND USE OF ATRARIC ACID AND THE DERIVATIVES THEREOF FOR THE TREATMENT OF PROSTATE CARCINOMA.
ISOLATION DE L'ACIDE ATRARIQUE, SYNTHESE DE DERIVES DE L'ACIDE ATRARIQUE, UTILISATION DE L'ACIDE ATRARIQUE ET DE SES DERIVES POUR LE TRAITEMENT DU CARCINOME DE LA PROSTATE

(30) Priorität: 05.02.2005 DE 102005005399
(43) Veröffentlichungstag der Anmeldung: 07.11.2007
(73) Patentinhaber: LTS LOHMANN Therapie-Systeme AG, 56626 Andernach (DE)
(72) Erfinder: HOFFMANN, Hans-Reiner, 56566 Neuwied (DE); MATUSCH, Rudolf, 35041 Marburg (DE); BANIAHMAD, Aria, 35043 Marburg (DE)
(74) Vertreter: Flaccus, Rolf-Dieter
(86) Internationale Anmeldenummer: PCT/EP2006/000749
(87) Internationale Veröffentlichungsnummer: WO 2006/081997

(56) Entgegenhaltungen:
- GB-A- 1 177 645
- US-A1- 2004 198 815
- A. ISHANI ET AL.: "Pygeum africanum for the Treatment of Patients with Benign Prostatic Hyperplasia: A Systematic Review and Qantitative Meta-analysis" THE AMERICAN JOURNAL OF MEDICINE, Bd. 109, Nr. 8, 2000, Seiten 654-664, XP002381913
- DATABASE MEDLINE [Online] US NATIONAL LIBRARY OF MEDICINE (NLM), BETHESDA, MD, US; 1. November 2000 (2000-11-01), SOLANO R M ET AL: "Effects of Pygeum africanum extract (Tadenan) on vasoactive intestinal peptide receptors, G proteins, and adenylyl cyclase in rat ventral prostate." XP002381933 Database accession no. NLM11074527 & THE PROSTATE. 1 NOV 2000, Bd. 45, Nr. 3, 1. November 2000 (2000-11-01), Seiten 245-252, ISSN: 0270-4137
- DATABASE CAPLUS [Online] CHEMICAL ABSTRACTS SERVICE, COLUMBUS, OHIO, US; SUN, HANDONG ET AL: "Chemical constituents of four medicinal lichens" XP002381934 gefunden im STN Database accession no. 1991:234925 & ZHIWU XUEBAO , 32(10), 783-8 CODEN: CHWHAY; ISSN: 0577-7496, 1990,
- DATABASE DATABASE CROSSFIRE Beilstein Institut zur Förderung der Chemischen Wissenschaften, Frankfurt am Main (DE); XP002381935 Database accession no. BRN 3279756 & FUJIKAWA: YAKUGAKU ZASHI, Bd. 60, 1940, Seite 473, 477,
- DATABASE CROSSFIRE BEILSTEIN Beilstein Institut zur Förderung der Chemischen Wissenschaften, Frankfurt am Main (DE); XP002381936 Database accession no. BRN 2099269 & FUJIKAWA: YAKUGAKU ZASSHI, Bd. 56, 1936, Seite 237, 245,
- DATABASE CROSSFIRE BEILSTEIN Beilstein Institut zur Förderung der Chemischen Wissenschaften, Frankfurt am Main (DE); XP002381937 Database accession no. BRN 2937096 & HERZIG ET AL.: MONATSH. CHEM., 24, 1903, Seite 908,
- DATABASE CROSSFIRE BEILSTEIN Beilstein Institut zur Förderung der Chemischen Wissenschaften, Frankfurt am Main (DE); XP002381938 Database accession no. BRN 2370614 & ROBERTSON; STEPHENSON: J. CHEM. SOC., 1930, Seite 313, 318,
- K. M. STRONG: "African Plum and Benign Prostatic Hypertrophy" JOURNAL OF HERBAL PHARMACOTHERAPY, Bd. 4, Nr. 1, 2004, Seiten 41-46, XP009066555
- SONJA SCHLEICH: "Nichtsteroidale Antiandrogene natürlichen Ursprungs zur Behandlung des Prostatakarzinoms" DISSERTATION, 2005, XP002381915 Gefunden im Internet: URL:http://archiv.ub.uni-marburg.de/diss/z 2005/0223/pdf/dss.pdf> [gefunden am 2006-05-16]

## Beschreibung

Die vorliegende Erfindung betrifft die Verwendung von Atrarsäure und ihrer Derivate zur Herstellung eines Arzneimittels zur Behandlung des Prostatakarzinoms, insbesondere des therapieresistenten Prostatakarzinoms.

Das Prostatakarzinom ist die häufigste Krebserkrankung unter Männern in der westlichen Welt und stellt nach Lungenkrebs die zweithäufigste zum Tode führende Krebsart dar. Obwohl das Prostatakarzinom in seiner Ätiologie nicht direkt mit der benignen Prostatahyperplasie (BPH) verknüpft ist, zeigen Patienten mit einer schweren Form der BPH sehr ähnliche Genanomalien wie Prostatakrebspatienten. Während die BPH vor allem die Transitionalzone der Prostata befällt, tritt ein Karzinom bevorzugt in der peripheren Zone auf.

Die Ursachen für die Entstehung eines Prostatakarzinoms liegen in diversen Gendefekten, die familiär prädisponiert vorliegen können. So kommen bei Erkrankten unter anderem diverse Mutationen des Androgenrezeptors vor. Eine reduzierte Aktivität der 5 α-Reduktase Typ II hingegen verringert das Risiko zur Entwicklung eines Karzinoms. Des Weiteren können verschiedene Tumorsuppressorgene wie z.B. Rb Gen auf Chromosom 13 q von Mutationen betroffen sein und somit inaktiviert werden. Auf der anderen Seite trägt eine Überfunktion an Onkogenen zur Tumorentstehung bei. Eine große Rolle spielen außerdem Methylierungen von wichtigen wachstumsregulierenden und detoxifizierenden Genen, die so funktionsunfähig werden und dem Krebs den Weg ebnen. Einen großen Beitrag leisten nach neuestem Stand der Wissenschaft auch Entzündungsprozesse, aus denen präneoplastische oder neoplastische Läsionen hervorgehen.

Die Anfangstherapie zur Behandlung eines Prostatakarzinoms besteht gewöhnlich in einer Entfernung der Prostata durch radikale Prostatektomie oder einer Bestrahlung, um die entarteten Zellen zu entfernen. Das fortgeschrittene, metastasierende Prostatakarzinom lässt sich durch eine lindernde Hormontherapie behandeln. Die heutzutage durchgeführte totale Androgenblockade beinhaltet die Kombination aus operativer und chemischer Kastration. Die reinen Antiandrogene Bicalutamid (Casodex®), Flutamid (Fugerel®) und Nilutamid (Anandron®) wirken selektiv auf die Androgenrezeptoren der Targetorgane, während Cyproteronacetat (Androcur®) auch Progesteron- und Glucocorticoidrezeptoren besetzt. Allerdings kann die Hormontherapie den fortgeschrittenen Prostatakrebs nicht heilen. Die Behandlung bewirkt zunächst eine antiandrogenabhängige Hemmung des Tumorwachstums. Nach durchschnittlich zwei Jahren tritt allerdings eine Therapieresistenz ein. Zunächst ermöglicht eine Überexpression von verschiedenen Coaktivatoren die Aktivierung des Androgenrezeptors durch nichtandrogene Steroide. Später können sogar Antiandrogene wie der aktive Flutamid-Metabolit 2-Hydroxyflutamid den Androgenrezeptor aktivieren und der Tumor wird androgenunabhängig.

ISHANI A et al ("Pygeum africanum for the treatment of patients with benign prostatic hyperplasia: A systematic review and quantitative meta-analysis"; The American Journal of Medicine, Bd. 109, Nr. 8, 2000, S. 654-664) berichten über Studien, in denen die Wirksamkeit von *Pygeum-africanum*-Extrakten (wie Tadenan®) bei der Behandlung der benignen Prostatahyperplasie untersucht wurde. Über die pharmakologisch aktiven Wirkstoffe dieser Extrakte ist nichts bekannt.

SOLANO RM et al ("Effects of Pygeum africanum (Tadenan) on vasoactive intestinal peptide receptors, G proteins, and adenylyl cyclise in rat ventral prostate"; US National Library of Medicine(NLM), Bethesda, MD, USA; 1. November 2000 (2000-11-01), Database accession no. NLM 11074527) beschreibt tierexperimentellen Studien, aus denen gefolgert wurde, dass Tadenan® eine regulatorische Wirkung verschiedene prostatische Komponenten des Adenylylcyclasesystems sowie auf die Proteinkinase-C-vermittelte Signaltransduktion ausübt. Welche Wirkstoffe für diese regulatorischen Effekte verantwortlich sind, wird nicht erwähnt.

In GB-1-1177645 werden Extrakte aus Prunus-Arten (darunter auch *P. africana*) beschrieben, die bei Ratten eine Verkleinerung der Prostata bewirkten. Diese Wirkung soll durch ein in den Extrakten enthaltenes "sterolhaltiges Material" verursacht sein.

STRONG KM ("African plum and benign prostatic hypertrophy"; Journal of Herbal Pharmacotherapy, Bd. 4, Nr. 1, 2004, S. 41-46) betrifft die Verwerdung von *Pygeum-africanum-*Extrakten zur Behandlung der benignen Prostatahyperplasie. Der genaue Wirkmechanismus ist nicht bekannt; als mögliche Wirkstoffe werden Phytosterole (beta-Sitosterol, beta-Sitosteron), pentazyklische Triterpene, n-docosanol und Tetracosanol genannt. Als mögliche Wirkmechanismen werden die Hemmung der Prostaglandinbiosynthese und Entzündungshemmung genannt.

US 2004/0198815 A1 bezieht sich auf die Verwendung von Methyl-β-Orcinolcarboxylat (Atrarsäure) zur Behandlung von pathogenen Pilzinfektionen beim Menschen. Außerdem wurde gefunden, dass diese Verbindung, ebenso wie ein ethanolischer Flechten-Extrakt, die Proliferation von Krebs-Zellinien aus der Leber, dem Enddarm, dem Ovar und dem Mund hemmt.

SUN H et al ("Chemical constituents of four medicinal lichens"; Database CAPLUS, Chemical Abstracts Service, Columbus, Ohio, US; Database accession no. 1991:234925) erwähnt Methyl-β-Orcinolcarboxylat als Inhaltsstoff von in der Medizin verwendeten Flechten ist; Angaben zu therapeutischen Indikationen fehlen.

2,4-dihydroxy-3,6-dimethyl-Benzoesäure-isopropylester ist unter der Nummer 3279756 im Beilstein-Register aufgeführt (Database Crossfire; Beilstein Institut z. Förderung der Chem. Wissenschaften, Frankfurt a.M. (DE), Database accession no. BRN 3279756).

2,4-dihydroxy-3,6-dimethyl-Benzoesäure-methylester (Methyl-β-Orcinolcarboxylat, Atrarsäure) ist unter der Nummer 2099269 im Beilstein-Register aufgeführt (Database Crossfire; Beilstein Institut z. Förderung der Chem. Wissenschaften, Frankfurt a.M. (DE), Database accession no. BRN 2099269). Die Verbindung wurde aus Flechten sowie aus der Borke von Bäumen isoliert. Als pharmaologische Wirkungen werden antibakterielle, antiproliferative, cytotoxische, nematozide und proteasehemmende Wirkungen angegeben.

2,4-dihydroxy-3-methyl-Benzoesäure-methylester ist unter der Nummer 2937096 im Beilstein-Register aufgeführt (Database Crossfire; Beilstein Institut z. Förderung der Chem. Wissenschaften, Frankfurt a.M. (DE), Database accession no. BRN 2937096).

2,4-dihydroxy-3,6-dimethyl-Benzoesäure-ethylester ist unter der Nummer 2370614 im Beilstein-Register aufgeführt (Database Crossfire; Beilstein Institut z. Förderung der Chem. Wissenschaften, Frankfurt a.M. (DE), Database accession no. BRN 2370614).

Die der vorliegenden Erfindung zugrunde liegende Aufgabe war es daher, neue Wirkstoffe zur Behandlung des Prostatakarzinoms zu finden, insbesondere Wirkstoffe, die auch androgenunabhängige Prostatakrebszellen in ihrem Wachstum hemmen.

Die der vorliegenden Erfindung zugrundeliegenden Aufgaben wurden dadurch gelöst, dass Substanzen mit antiandrogener Wirkung aus der Rinde des afrikanischen Pflaumenbaums *P.* africana isoliert wurden.

Überraschenderweise wurde die Substanz Atrarsäure aus der Rinde von *P. africana* oder aus auf der Rinde von *P. africana* wachsenden Flechten isoliert und gefunden, dass diese Substanz eine hohe antiandrogene Aktivität hat. Atrarsäure ist sogar in der Lage, das Wachstum von Prostatakrebszellen, die nicht auf eine Hydroxyflutamid-Behandlung ansprechen, zu inhibieren.
Darüber hinaus konnte nachgewiesen werden, dass Atrarsäure agonistisch auf die beiden Estrogenrezeptoren (Estrogenrezeptor alpha (ERα) und Estrogenrezeptor beta (ERβ)) wirkt.

Demnach betrifft die vorliegende Erfindung die Verwendung eines 2,4-Dihydroxy-3-methylbenzoats der allgemeinen Formel 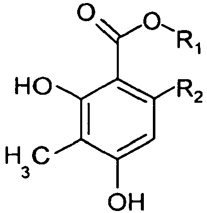 wobei R₁ C₁- bis C₄-Alkyl bedeutet und R₂ Wasserstoff oder ein Methyl-, Ethyl- oder Propylrest ist, zur Herstellung eines Arzneimittels zur Behandlung des Prostatakarzinoms.

*Prunus africana* (Hook. f.) Kalkm. mit dem obsoleten botanischen Bestimmungsnamen *Pygeum africanum* (Hook. f.) gehört zur Unterfamilie der Prunoideae innerhalb der Rosaceae. Zu den Prunoideae zählen Holzpflanzen mit Steinfrüchten. Die Gattung *Prunus* ist die Umfassendste in ihrer Unterfamilie, zu ihr gehören beispielsweise auch die Kirsche (*Prunus avium* L.), der Pfirsich (*Prunus persica* L.), die Pflaume (*Prunus domestica* L.) und die Mandel (*Prunus dulcis* (Mill.) D.A. Webb). Der afrikanische Pflaumenbaum *Prunus africana* (Hook. f.) Kalkm. kommt als einzige Art dieser Gattung auf dem afrikanischen Kontinent vor und sollte sich so bezüglich seiner Inhaltsstoffe von den anderen Vertretern aus derselben Unterfamilie unterscheiden.

Das Ziel der vorliegenden Arbeit war, die antiandrogen wirkenden Naturstoffe aus der Rinde von *Prunus africana* zu isolieren, da ein Extrakt mit einer Vielzahl an Inhaltsstoffen nur bei Kenntnis aller wirksamen Substanzen samt ihrer genauen Wirkstärke standardisierbar ist und außerdem eine höhere Belastung des Organismus bewirkt. Darüber hinaus sollten neue antiandrogene Wirkstoffe auf Basis der aus *P. africana* isolierten antiandrogen wirkenden Substanzen hergestellt werden.
FIG. 1 zeigt die Inhibition der Aktivität eines Androgens im Luciferase-Assay durch unterschiedliche Extrakte von *P*. *africana.*
FIG.2 stellt die antiandrogene Wirkung von Fraktionen eines selektiven Methylenchlorid-Extrakts von *P. africana* dar.
FIG. 3 veranschaulicht die antiandrogene Wirkung von aus Fraktion F8 des selektiven Methylenchlorid-Extrakts isolierten Verbindungen.
FIG. 4 verdeutlicht die Inhibition des Wachstums menschlicher Prostatakarzinomzellen durch Atrarsäure.
FIG. 5 ist eine Darstellung der Strukturformel von Atrarsäure (AA) und Atrarsäure-Derivaten (A1 bis A6).
FIG. 6 zeigt die Ergebnisse von Luciferase-Assays mit synthetisierten Strukturvarianten der Atrarsäure (AA) bei einer Konzentration von 10⁻⁶ M.
FIG. 7 veranschaulicht die antiandrogene Wirkung von Verbindungen mit strukturellen Ähnlichkeiten zu Atrarsäure.
FIG. 8 veranschaulicht die agonistische Wirkung von Atrarsäure auf den Estrogenrezeptor beta.
FIG. 9 veranschaulicht die agonistische Wirkung von Atrarsäure auf den Estrogenrezeptor alpha.

Zunächst wurde die antiandrogene Wirksamkeit von verschiedenen Pygeum-Extrakten miteinander verglichen. Mittels wirkungsorientierter Fraktionierung wurde dann die Isolierung der wirksamen Verbindungen vorgenommen. Zur gezielten Fraktionierung des Rindenmaterials von *P. africana* (Hook. f.) Kalkm. wurden zunächst selektive Extrakte hergestellt.

Im Allgemeinen zeichnen sich die Inhaltsstoffe von pflanzlichen Drogen durch ihr hohes Maß an Biodiversität aus, die sich in einer enormen Vielfalt an unterschiedlichsten Verbindungen äußert. Um trotzdem Extrakte mit einer überschaubaren Anzahl an Inhaltsstoffen zu erhalten, hat es sich bewährt, eine Vorfraktionierung nach Löslichkeit in Lösungsmitteln steigender Polarität vorzunehmen. Die resultierenden selektiven Extrakte sind aufgrund der Einschränkung des Polaritätsbereiches und der Anreicherung von Substanzen geringerer Konzentration leichter chromatographisch zu handhaben und ermöglichen so nach weiterer Fraktionierung schließlich die Isolierung von wirksamen Inhaltsstoffen.

In der vorliegenden Arbeit wurde die Prozedur der selektiven Extraktion zweimal vorgenommen. Das zerkleinerte Pflanzenmaterial wurde gesiebt, anschließend in *n*-Hexan mit einem Ultra-Turrax weiter zerkleinert und schließlich in eine beidseitig mit Stahlfritten verschlossene Edelstahlkartusche (40 x 10 cm bzw. 80 x 10 cm) gefüllt. Mittels einer HPLC-Pumpe wurden die Lösungsmittel nach steigender Polarität (n-Hexan, Dichlormethan, Methanol, Methanol/Wasser (50/50) und Wasser) durch die befüllte Kartusche befördert. Die so erfolgte Extraktion wurde jeweils bis zur Erschöpfung vorgenommen und die gewonnenen Extrakte wurden anschließend unter vermindertem Druck zur Trockne eingeengt. Dieses Extraktionsverfahren ist äußerst schonend, sowohl Temperaturbelastung als auch direkte Sauerstoff- und Lichteinwirkung auf die Droge werden so vermieden. Wesentlich ist aber, dass dadurch die Inhaltsstoffe in nach Polarität geordneten Extrakten vorsortiert sind und so besser chromatographiert werden können.

Betrachtet man die Massenanteile der selektiven Extrakte an der Gesamtextraktmenge, so wird deutlich, dass das Pflanzenmaterial überwiegend Methanol-lösliche Bestandteile enthält. Die lipophilen Extrakte aus *n*-Hexan und Dichlormethan fallen weniger gewichtig aus. Im Allgemeinen befinden sich im Hexanextrakt Harze, Öle, Fette oder fettähnliche Substanzen, die langkettigen Alkohole und Fettsäuren aus *P. africana* müssten also hier zu finden sein. Die in *P. africana* vorkommenden Phytosterole und pentacyclischen Triterpene lassen sich im Dichlormethanextrakt vermuten. Stark polare Pflanzeninhaltsstoffe wie Aminosäuren, anorganische Salze oder Saccharide hingegen werden erst mit Methanol/Wasser oder Wasser extrahiert.

Neben den selektiven Extrakten wurde auch ein ethanolischer Gesamtextrakt aus der Rinde von *P*. *africana* (Hook. f.) Kalkm. hergestellt. Hierzu wurden 300 g des gesiebten Pflanzenmaterials in absolutem Ethanol mit einem Ultra-Turrax weiter zerkleinert und in mehreren Portionen mit insgesamt 5 1 Ethanol extrahiert. Anschließend wurde der Extrakt filtriert und schließlich unter vermindertem Druck bis zur Trockne eingeengt.

Die potentiell antiandrogene Wirkung der gewonnenen Extrakte wurde anhand des Androgenrezeptor-abhängigen MMTV-luc Reportergen-Assays, im Folgenden kurz mit Luciferase-Assay bezeichnet, untersucht.

Bei diesem Assay dient das Enzym Luciferase als Reportergen. Die Luciferase ist eine Oxidoreduktase aus dem nordamerikanischen Leuchtkäfer *Photinus pyralis,* die das Substrat Luciferin in Anwesenheit von Luftsauerstoff, ATP und Mg²⁺-Ionen zu Oxyluciferin dehydriert, die dabei freiwerdende Energie wird als Licht emittiert.

Das Reportergen Luciferase befindet sich auf dem Plasmid pMMTV-luc (MMTV = Mouse mammary tumor virus), auf welchem außerdem das Androgen responsive Element (ARE) lokalisiert ist. Das Plasmid pMMTV-luc wird zusammen mit dem Adrenogenrezeptor-Expressionsvektor in Fibroblasten der Affenniere transfiziert. Wird nun ein Androgen hinzugegeben, bindet dieses im Komplex mit dem Androgenrezeptor an das Androgen responsive Element. Dieser Vorgang initiiert nun die Transkription des nachfolgenden Gens, nämlich die des Luciferase-Reportergens. Die Menge an exprimierter Luciferase ist direkt proportional zur bei Hinzugabe von Luciferin freiwerdenden Lichtmenge, die über die Messung der Emission bei λ = 562 nm quantifiziert werden kann. Ist nun neben dem Androgen auch eine antiandrogene Substanz oder ein antiandrogener Extrakt präsent, ist die Transaktivierung des Luciferase-Reportergens durch das Androgen responsive Element gehemmt, es wird nachfolgend bei Substratzugabe entsprechend weniger Lichtenergie frei. Da die Abnahme an Lichtmenge direkt proportional zur Hemmwirkung des Antiandrogens ist, eignet sich dieser Assay hervorragend für die Suche nach neuen antiandrogenen Leitstrukturen.

Die Extrakte wurden nun mit dem Luciferase-Assay in zwei Konzentrationen (300 µg/ml und 600 µg/ml) auf ihre antiandrogene Bioaktivität untersucht. Die antiandrogene Wirkung wurde zur Auswertung als prozentuale Inhibition gegenüber einer Kontrolle berechnet, bei der lediglich reines Lösungsmittel zugesetzt wurde. Der wirksamste Extrakt sollte dann einer weiteren wirkungsorientierten Fraktionierung unterzogen werden.

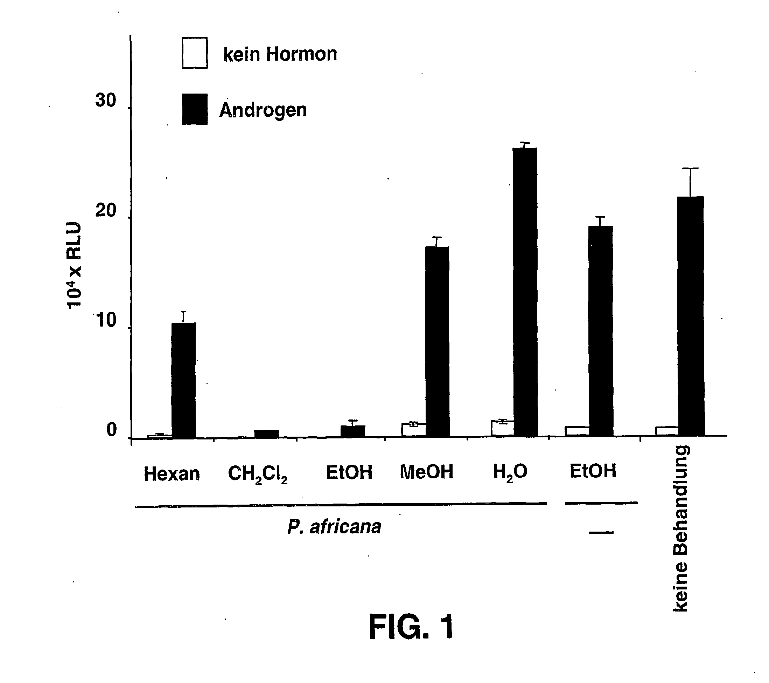

Figur 1 zeigt, dass der selektive Hexanextrakt aus *P*. *africana* eine schwache antiandrogene Wirkung aufweist, die vermutlich auf den hohen Gehalt an freien und veresterten Fettsäuren und langkettigen Alkoholen zurückzuführen ist.

Der selektive Dichlormethanextrakt aus *P*. *africana* offenbarte im Assay den größten antiandrogenen Effekt, dieser Extrakt wurde daher für die weitere wirkungsorientierte Fraktionierung ausgewählt.

Mit zunehmender Hydrophilie der selektiven Pygeum-Extrakte nimmt die antiandrogene Wirkung deutlich ab.

Der ethanolische Gesamtextrakt aus *P*. *africana* zeigt wiederum einen starken Effekt. Das deutet darauf hin, dass mit Ethanol die gleichen antiandrogenen Substanzen extrahiert wurden wie im selektiven Dichlormethanextrakt. Dieser ist noch wirksamer, da hier die Anreicherung der Wirksubstanzen gelang.

Bei der Suche nach der aktiven Verbindung aus einem komplexen Gemisch zahlreicher Substanzen hat sich die Methode der wirkungsorientierten Fraktionierung bewährt. Hierzu wird der pflanzliche Extrakt zunächst auf seine biologische Wirkung hin getestet. Beim Auftreten eines Effektes wird die Probe chromatographisch aufgetrennt und alle Fraktionen erneut auf Wirksamkeit getestet. In der Regel ist die Wirkung nicht über alle Fraktionen verteilt, sondern findet sich in wenigen klar definierten wieder, da hier eine Anreicherung der aktiven Substanzen stattgefunden hat. Die aktiven Fraktionen werden dann ausgewählt und mit einem anderen Trennverfahren weiter fraktioniert. Dieses Vorgehen wird mit immer spezifischeren Trennmethoden so oft wiederholt, bis man schließlich die aktiven Substanzen isoliert hat. Die Kombination von unterschiedlichen Trennverfahren (selektive Extraktion, Ausschütteln, Normalphasen- und Umkehrphasen-Chromatographie usw.) reduziert die Anzahl an Fraktionierungsschritten und damit den zeitlichen Aufwand. Die isolierten Substanzen werden dann mit verschiedenen analytischen Methoden identifiziert und quantifiziert und schließlich als Einzelsubstanz und in Mischungen aller aktiven Verbindungen auf ihre Wirksamkeit getestet. Besteht Übereinstimmung zwischen einer Referenzsubstanz und der aus dem Extrakt isolierten Verbindung, so gelten die identifizierten Substanzen als bestätigt.

In der vorliegenden Arbeit wurde ebenso verfahren. Zunächst wurde der Extrakt mit der höchsten Wirksamkeit ausgewählt. Dieses war der selektive Dichlormethanextrakt aus *P*. *africana,* der aufgrund der Vorgehensweise der selektiven Extraktion schon vorfraktioniert ist. Die weitere Fraktionierung des Extraktes erfolgte mit Gradientenextrographie, einer Chromatographie auf Normalphase.

Das Verfahren der Extrographie wurde für die Fraktionierung von Erdöldestillationsrückständen entwickelt. Es dient der Auftrennung von komplexen Gemischen, deren Inhaltstoffe einen breiten Polaritätsbereich umfassen. Das komplexe Gemisch wird mit einem groben Stufengradienten in eine überschaubare Anzahl an Fraktionen mit jeweils engerem Polaritätsbereich getrennt. Dies hat eine Anreicherung von Substanzen bestimmter Polarität in der jeweiligen Fraktion zur Folge. Dieses Verfahren wurde für die Auftrennung von pflanzlichen Extrakten modifiziert, wobei insbesondere die Probenzone auf wenige Zentimeter verkürzt wurde. Auf diese Weise können in relativ kurzer Zeit große Mengen an Extrakt fraktioniert werden.

Bei der Extrographie wird zunächst der Extrakt in einem geeigneten Lösungsmittel gelöst und auf die etwa fünffache Menge an grobem Kieselgel aufgezogen. Hierzu vereinigt man das Kieselgel mit der klaren Extraktlösung, behandelt diesen Ansatz mit Ultraschall und entfernt anschließend das Lösungsmittel am Rotationsverdampfer unter langsamem Drehen des Kolbens bis ein trockenes, rieselfähiges Material zurückbleibt. Dieser Vorgang bewirkt eine Vorsortierung der Probenmoleküle auf dem Kieselgel. Die äußerst polaren Silanolgruppen des Kieselgels adsorbieren an ihrer Oberfläche zunächst die Probenmoleküle höchster Polarität. Diese neue Oberfläche aus polaren Verbindungen adsorbiert wiederum etwas weniger polare Substanzen aus dem Extrakt. Es entstehen so mehrere Schichten aus Probenmolekülen abnehmender Polarität in den Kieselgelporen. An der neuen Porenoberfläche befinden sich also die apolarsten Substanzen. Daraus ergibt sich auch zwingend, dass der gelöste Extrakt in einer einzigen Portion vollständig auf das Kieselgel gegeben werden muss und nicht portionsweise, da dann bei jeder Portion nach Abzug des Lösungsmittels wieder alle Polaritäten der Substanzen neu aufgezogen würden. Das Kieselgel mit dem aufgezogenen Extrakt wird in die Trennsäule vor das chromatographische Bett aus feinem Kieselgel (Macherey - Nagel Si60, 15-25 µm) gepackt. Beginnt man dann den Lösungsmittelgradienten mit einem lipophilen Eluenten, werden zunächst nur die lipophilen Substanzen an der Oberfläche gelöst und dem chromatographischen Trennbett zugeführt. Im Verlauf des Gradienten steigert man die Polarität des Eluenten, so dass nun auch immer stärker polare Substanzen der Chromatographie zur Verfügung gestellt werden. Die Vorsortierung der Probenmoleküle auf dem Kieselgel ermöglicht somit eine Fraktionierung großer Substanzmengen. Einer Überladung des Trennbettes wird vorgebeugt, da die Probenmoleküle nicht auf einmal, sondern nach und nach in Gruppen unterschiedlicher Polarität geordnet ins Trennbett gelangen.

Der für den selektiven Dichlormethanextrakt am besten geeignete Lösungsmittelgradient wurde anhand einer Reihe an Vorversuchen mit verschiedenen Gradienten ermittelt. Als lipophilste Lösungsmittelkomponente wurde wie bei der selektiven Extraktion n-Hexan gewählt. Im weiteren Verlauf des Gradienten sollte langsam und gleichmäßig Dichlormethan hinzugemischt werden, da eine Auftrennung des Extraktes, der ja auch mit diesem Lösungsmittel hergestellt wurde, damit am besten erfolgen müsste. Anschließend erfolgt das Beimischen von Methanol und schließlich das von Wasser in relativ kurzer Zeit, da derart polare Verbindungen im Dichlormethanextrakt kaum zu vermuten sind. Zu allen Eluenten wurde 0,1 % Trifluoressigsäure hinzugesetzt, um saure Verbindungen an ihrer Dissoziation zu hindern.

Die Extrographie wurde im präparativen Maßstab zweimal mit dem selektiven Dichlormethanextrakt aus *P*. *africana* durchgeführt (Extrographie 1 = E1 und Extrographie 2 = E2). Das Upscaling in den Großmaßstab erforderte die Anpassung der Säulendimension und der Flussrate des Elutionsmittels. Als Säule diente eine mit Kieselgel befüllte Edelstahlkartusche (Merck Prepbar® 40 x 10 cm), deren Füllung mit Hilfe eines Presswerkzeuges und eines variablen Säulenkopfes verdichtet wurde. Die Probenzone, bestehend aus dem aufgezogenen Extrakt, befand sich unten im Chromatographierohr. Die Eluenten wurden mit einer Flussrate von 120 ml/min mit einer HPLC-Pumpe von unten nach oben gepumpt, um Lufteinschlüsse zu vermeiden.

Die bei der präparativen Gradientenextrographie erhaltenen 35 Fraktionen (Tabelle 1) wurden HPLC-analytisch auf ihre Zusammensetzung untersucht und untereinander und mit dem unfraktionierten selektiven Dichlormethanextrakt verglichen. Dabei wurden alle Fraktionen in gleichen Mengen chromatographiert, so dass ein direkter Konzentrationsvergleich jeweils gleicher Verbindungen in den einzelnen Fraktionen anhand der UV-Absorption und damit der Flächen im Chromatogramm vorgenommen werden konnte.

Vergleicht man die Chromatogramane der Extrographiefraktionen mit dem Übersichtschromatogramm, erkennt man deutlich die mit der Extrographie geglückte stoffliche Abtrennung der Inhaltsstoffe voneinander. Einige Substanzen reicherten sich in den entsprechenden Fraktionen stark an.

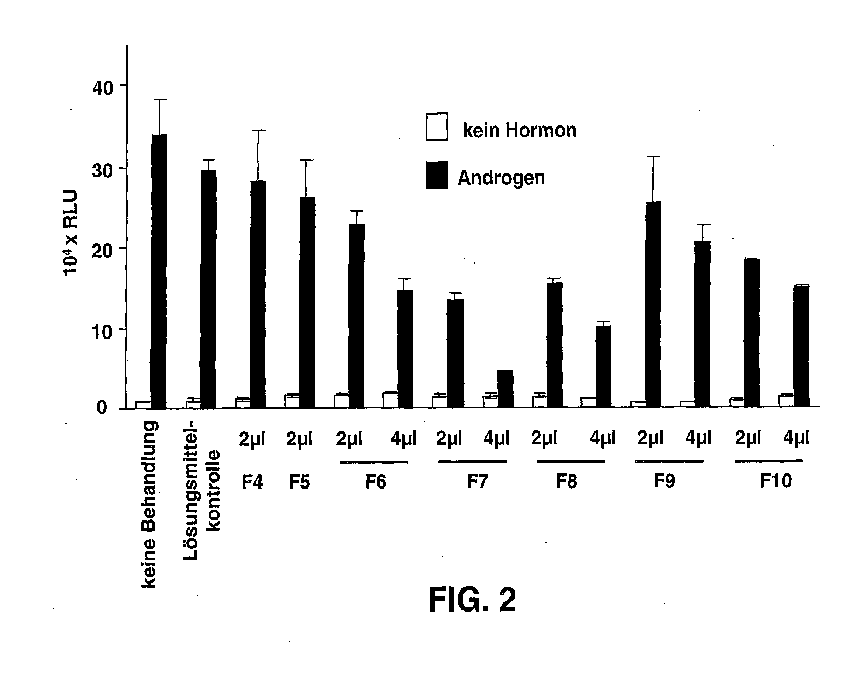

Die 35 Fraktionen wurden dem Luciferase-Assay zum Test auf antiandrogene Wirkung zugeführt. Alle Fraktionen wurden in den Konzentrationen 30 µg/ml und 60 µg/ml getestet. Von den 35 Fraktionen erwiesen sich drei als äußerst wirksam, nämlich die benachbarten Fraktionen F6, F7 und F8, wie aus Figur 2 zu ersehen ist.

Beim Vergleich der HPLC-Chromatogramme der Fraktionen F6, F7 und F8 fiel auf, dass alle drei Fraktionen ein sehr ähnliches Profil an Inhaltsstoffen aufweisen. Die Anzahl an Peaks hat sich im Vergleich zum unfraktionierten selektiven Dichlormethanextrakt deutlich reduziert. Besonders ein Doppelpeak bei einer Retentionszeit von 39 Minuten stach ins Auge, da er in allen anderen 32 Fraktionen nicht gefunden wird. Diese Tatsache erweckt die Vermutung, dass es sich bei einer der beiden oder sogar beiden Substanzen, die sich unter dem Doppelpeak verbergen, um die wirksamen Komponenten aus *P*. *africana* handeln könnte.

Zur Gewinnung der antiandrogenen Wirkstoffe stand nun die letzte Trennstufe an, nämlich die weitere Isolierung aus den antiandrogen wirksamen Extrographiefraktionen.

Von den drei wirksamen Extrographiefraktionen F6, F7 und F8 wurde für die weitere präparative Auftrennung die in ausreichender Menge vorhandene Fraktion F8 ausgewählt. Die analytische Trennmethode wurde gekürzt und durch Anpassung der Säulendimension und der Flussrate in den präparativen Maßstab überführt. Nach mehreren Trennungen konnten die Substanzen P3, P5, P7, P9 und P10 in für den Luciferase-Assay ausreichender Menge erhalten werden.

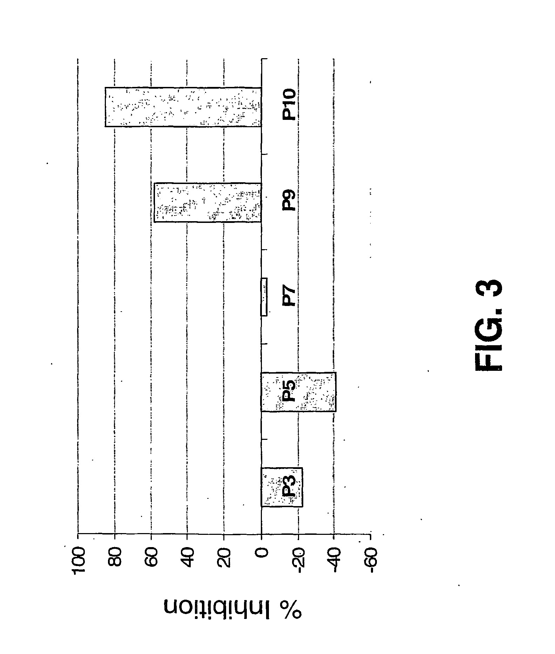

Die isolierten Substanzen P3, P5, P7, P9 und P10 wurden mittels Luciferase-Assay auf ihre antiandrogene Wirkung getestet. Die Ergebnisse sind in Figur 3 dargestellt.

Aus Figur 3 geht deutlich hervor, dass es sich bei den Substanzen P9 und P10 um die antiandrogen wirksamen Verbindungen aus *P. africana* handelt. Verbindung P5 zeigte im Luciferase-Assay eine moderate androgene Wirkung, P3 erwies sich als schwach androgen und P7 ließ keinen signifikanten Effekt erkennen.

Vor der präparativen Trennung vow F8 wurde die Fraktion in dem Lösungsmittelgemisch der chromatographischen Anfangsbedingungen gelöst (20% Acetonitril (ACN), 80% Wasser). Es blieb ein Rückstand, der abfiltriert und durch Lösen in Ethanol/DMSO auch getestet werden konnte. Der Rückstand zeigte im Luciferase-Assay keinen Effekt.

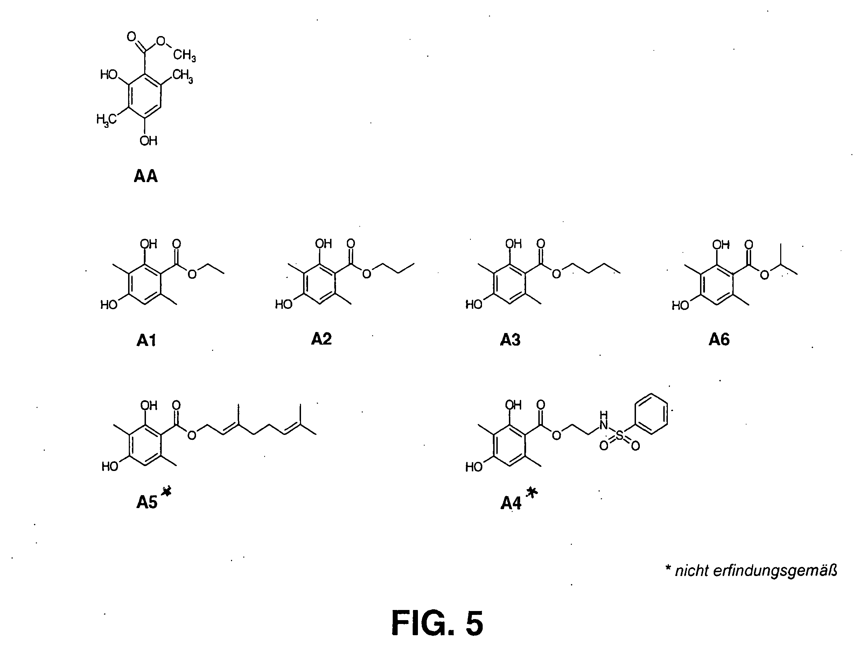

Zur Strukturaufklärung der Substanz P9 dienten die Aufnahmen von ¹H-NMR-, ¹³C-NMR-, UV-, IR- und EI-MS-Spektren. P9 konnte als Methyl-2,4-dihydroxy-3,6-dimethylbenzoat, das auch als Methyl-β-Orcinolcarboxylat bezeichnet wird, mit dem Trivialnamen Atrarsäure identifiziert werden. Die Bezeichnung "Säure" ist etwas irreführend, da die Carbonsäure-Funktion von Atrarsäure nicht frei vorliegt, sondern mit Methanol verestert ist. Dennoch hat Atrarsäure aufgrund der beiden phenolischen Hydroxyl-Gruppen und der phenylogen Carbonyl-Gruppe saure Eigenschaften. Die Strukturformel von Atrarsäure (AA) ist in Figur 5 dargestellt.

Atrarsäure wurde als farblose Nadeln aus Fraktion F8 isoliert. Die Substanz hat einen charakteristischen holzigen Geruch. Das IR-Spektrum einer Lösung von Atrarsäure in Chloroform zeigt neben den Absorptionsbanden für die Methoxycarbonyl-Gruppe zwei Banden für die beiden Hydroxyl-Gruppen. Die OH-Valenzschwingung bei vₘₐₓ= 3040 cm⁻¹ deutet auf das Vorliegen einer intramolekularen Wasserstoffbrücke hin, die das Molekül zwischen der Hydroxyl-Gruppe an Position C-2 und der Carbonyl-Gruppe ausbildet. Bei Verdünnung der Lösung bleibt die Bande in gleicher Lage bestehen. Die Absorptionsbande der OH-Valenzschwingung bei vₘₐₓ = 3400 cm⁻¹ verschiebt sich bei Verdünnung der Probe allerdings zu größeren Wellenzahlen, was ein Hinweis für das Vorliegen einer intermolekularen Wasserstoffbrücke ist. Die Verdünnung bewirkt ein Aufbrechen der intermolekularen Wasserstoffbrücke zwischen der Hydroxyl-Gruppe an C-4 und der Carbonyl-Gruppe eines zweiten Moleküls, wodurch sich die Bindungsstärke der OH-Gruppe erhöht und somit ein höherer Energiebetrag zur Anregung der Valenzschwingung nötig ist.

Die intramolekulare Wasserstoffbrücke lässt sich auch im ¹H-NMR-Spektrum erkennen. Das Signal des Protons der an der intramolekularen Wasserstoffbrücke beteiligten Hydroxyl-Gruppe an C-2 ist ungewöhnlich scharf und stark ins tiefe Feld verschoben (δ = 11,98 ppm). Bei einem HD-Tausch wird dieses Proton nicht in dem Maße gegen Deuterium ausgetauscht wie das Proton der anderen Hydroxyl-Gruppe an C-4. Diese Tatsache verdeutlicht, dass die intramolekulare Wasserstoffbrücke deutlich stärker sein muss als die intermolekulare. 1983 gelang die Aufnahme der Kristallstruktur der Atrarsäure.

Das ¹H-NMR-Spektrum der Atrarsäure weist sechs Singuletts auf. Die Signale der Methyl-Gruppen befinden sich bei Verschiebungen von δ = 2,03 ppm und δ = 2,39 ppm. Ein Singulett mit der Signalintensität von drei Protonen bei der Verschiebung von δ = 3,85 ppm deutet auf eine Methoxycarbonyl-Gruppe hin, was die Signale im "C-NMR-Spektrum bei δ = 51,8 ppm und δ = 172,6 ppm bestätigen. Das ¹³C-NMR-Spektrum zeigt eine extrem hochfeldige Verschiebung des Signals für die Methyl-Gruppe an C-3 bei δ = 7,6 ppm, was sich mit den stark elektronenziehenden Effekten der beiden Hydroxyl-Gruppen an den Nachbar-C-Atomen erklären lässt. Diese Position der Methyl-Gruppe ließ sich mit einem HMBC-Experiment (HMBC = Heteronuclear Multiple Bond Correlation) bestätigen, welches im Spektrum ein Kreuzsignal für die ³J(C,H)-Kopplung zwischen den Methyl-Protonen an C-3 mit C-2 und C-4 aufweist.

Die Zuordnung aller Protonen zu den entsprechenden Kohlenstoffatomen wurde durch die Aufnahme eines HMQC-Spektrums (HMQC = Heteronuclear Multiple Quantum Coherence) realisiert.

Das EI-Massenspektrum von Atrarsäure ergibt einen Molekülionenpeak bei m/z 196. Die Summenformel C₁₀H₁₂O₄ konnte durch die Massenfeinbestimmung bestätigt werden. Das Spektrum zeigt außerdem die beiden charakteristischen Fragmentionenpeaks der Atrarsäure. Das Fragmention bei m/z 164 entsteht durch die Abspaltung von Methanol, bei der die Methyl-Gruppe der Methoxycarbonyl-Gruppe mit dem Proton der Hydroxyl-Gruppe an C-2 Methanol bildet und als dieses abgespalten wird. Dies ist charakteristisch für eine Methylsalicylat-Partialstruktur. Eine weitere Fragmentierung bewirkt die Abspaltung von Kohlenmonoxid, es entsteht der zweite Fragmentionenpeak bei *m*/*z* 136.

Das UV-Spektrum von Atrarsäure zeigt drei Maxima bei λ = 217, 245 und 307 nm. Alkalische Bedingungen bewirken eine Gelbfärbung der Lösung und damit eine bathochrome Verschiebung der Maxima im W-Spektrum.

Es ist möglich, Atrarsäure aus Aceton umzukristallisieren, um monokline Kristalle zu erhalten. Außerdem gibt Atrarsäure aufgrund der Salicylat-Partialstruktur eine violette Färbung mit Eisen(III)chlorid-Lösung. Alle analytischen Daten befinden sich in guter Übereinstimmung mit Literaturwerten.

Zur Gehaltsbestimmung von Atrarsäure im selektiven Dichlormethanextrakt aus *P. africana* wurde eine Eichgerade mit einer Referenzsubstanz von Atrarsäure mit einer Reinheit von über 99 % erstellt. Es ergab sich ein Gehalt von 0,16 % (m/m) von Atrarsäure im selektiven Dichlormethanextrakt.

Atrarsäure weist im Luciferase-Assay eine deutliche antiandrogene Wirkung auf.

Atrarsäure wurde bereits aus dem Rindenmaterial von verschiedenen höheren Pflanzen isoliert, wie z.B. *Newbouldia laevis, Alseodaphne andersonii, Acer nikoense, Xylosma velutina* und *Ekebergia pterophylla.*

Atrarsäure ist aber außerdem als Flechteninhaltsstoff bekannt. Flechten sind Epiphyten, d.h. symbiotische Organismen, die aus Pilzen (Mycobiont) und Algen (Photobiont) bestehen. Atrarsäure kann in Flechten frei vorliegen und außerdem dient sie als Baustein von Depsiden und Depsidonen. Beispielsweise ist der bekannte Flechteninhaltsstoff Atranorin ein Depsid aus Atrarsäure und Hematomminsäure und wird von verschiedenen Lichen-Arten über den Polyketidstoffwechsel synthetisiert.

Diese Tatsache wirft die Frage auf, ob Atrarsäure tatsächlich ein sekundärer Metabolit von *Prunus africana* (Hook. f) Kalkm. ist oder ob die Rinde des Baumes von einer Flechte befallen war, die Atrarsäure als Polyketid über den Acetat-Polymalonatsyntheseweg gewinnt.

Bei der Betrachtung der Rindendroge von *Pygeum africanum* unter dem Mikroskop erkennt man deutlich Hyphen, die das Vorliegen einer Flechte bestätigen. Dies weist darauf hin, dass Atrarsäure aus dem Polyketidstoffwechsel der Flechte stammt und kein sekundärerer Pflanzenmetabolit von *Pygeum africanum* ist.

Atrarsäure zählt zu den Flechtensäuren, die Polyacetate sind und deren Biogenese durch die Pilzkomponente des Pflanzenthallus erfolgt. Atrarsäure gilt wie andere Flechtensäuren auch als antimikrobiell und nematozid.

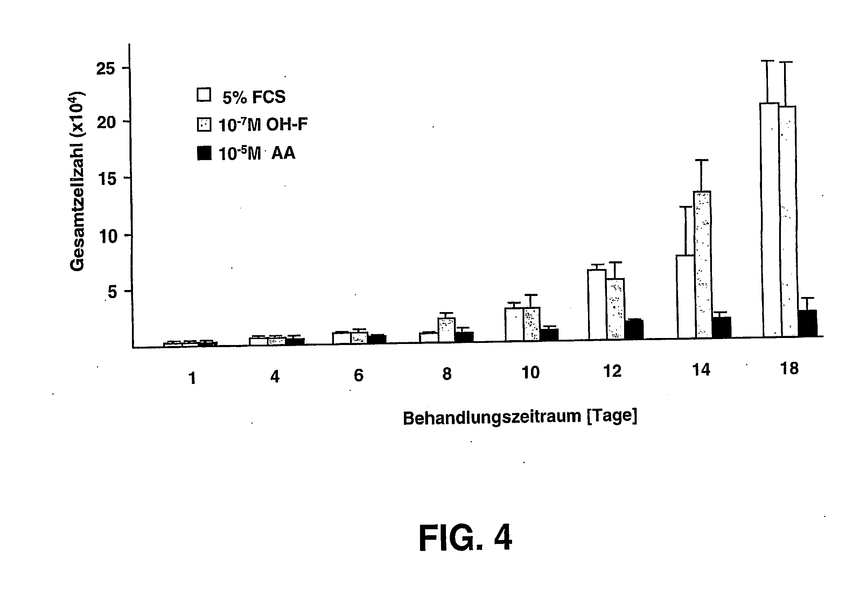

Atrarsäure kann mittlerweile auch vollsynthetisch aus 3-Methyl-4-methylen-2-oxetan und Acetessigsäuremethylester hergestellt werden.
Das Wachstum von Prostatazellen und Prostatakrebszellen ist ursprünglich abhängig von Androgenen. Um zu untersuchen, ob der Androgen-Antagonismus von Atrarsäure auch das Zellwachstum beeinträchtigt, wurde die menschliche Prostata-Krebszelllinie LNCaP verwendet, von der bekannt ist, dass sie Androgen-abhängig wächst. LNCaP-Zellen wurden in Kultur in Anwesenheit von 10 µM Atrarsäure kultiviert. Figur 4 zeigt, dass die Zellen, die mit 10 µM Atrarsäure behandelt wurden, bereits am 8. Behandlungstag ein deutlich geringeres Wachstum zeigten als die unbehandelten Zellen. Dieser Effekt wurde bis Tag 18 der Behandlung noch deutlicher. In Gegenwart von 10 µM Atrarsäure zeigten die LNCaP Zellen ein verringertes Wachstum, während die Behandlung mit OH-F (Hydroxyflutamid) zu keiner Wachstumsverringerung führte. Letzteres könnte dadurch bedingt sein, dass LNCaP Zellen eine Punktmutation in der Ligandbindungsdomäne des menschlichen Androgenrezeptors haben, die verhindert, dass OH-F antiandrogen in diesen Zellen wirkt.

Diese Ergebnisse zeigen, dass der Androgen-Antagonsimus von Atrarsäure auch bei einem mutierten menschlichen Androgen-Rezeptor wirksam ist. Somit ist Atrarsäure in der Lage, das Wachstum von LNCaP Zellen zu inhibieren. Daher könnte Atrarsäure auch zur Behandlung von Prostatakarzinomen verwendet werden, die resistent gegen bekannte antiandrogene Wirkstoffe wie Hydroxyflutamid sind.

Die vorliegende Erfindung betrifft daher auch die Verwendung von Atrarsäure zur Herstellung eines Medikaments zur Behandlung des Prostatakarzinoms, insbesondere von Prostatakarzinomen, die resistent gegen eine Behandlung mit bekannten Androgen-Antagonisten wie beispielsweise Bicalutamid, Flutamid, Hydroxyflutamid, Nilutamid oder Cyproteronacetat sind.

Zur Strukturoptimierung von Atrarsäure wurde eine Reihe von Substanzen synthetisiert, die sich in der Ester-Gruppe von Atrarsäure unterscheiden. Hierzu wurde zunächst versucht, eine sauer katalysierte Veresterung von 2,4-Dihydroxy-3,5-dimethylbenzoesäure mit verschiedenen primären aliphatischen Alkoholen durchzuführen. Infolge der geringen Carbonylaktivität reagieren Carbonsäuren im Allgemeinen nur langsam mit Alkoholen. Der Zusatz von starken Mineralsäuren wie Schwefelsäure und das Refluxieren über mehrere Stunden kann die Reaktionsgeschwindigkeit erheblich beschleunigen. Die Reaktion von 2,4-Dihydroxy-3,5-dimethylbenzoesäure mit einem primären Alkohol wie beispielsweise Ethanol lieferte allerdings nicht den gewünschten Ester, da 2,4-Dihydroxy-3,5-dimethylbenzoesäure aufgrund ihrer Salicylat-Struktur in der Hitze mit Mineralsäuren decarboxyliert.

Allerdings lieferte eine alkalisch katalysierte Umesterung von Atrarsäure mit einem primären Alkohol den gewünschten Ester. Hierzu wurde Atrarsäure über Nacht mit etwas mehr als der äquimolaren Menge an Kaliumhydroxid in einer Lösung des entsprechenden primären aliphatischen Alkohols bzw. Benzolsulfonamid gerührt. Da diese Umesterung nicht quantitativ verläuft, musste das Reaktionsprodukt mittels präparativer HPLC aus dem Gemisch isoliert werden.

Auf diese Weise gelang die Synthese der folgenden Atrarsäure-Derivate (Atratate), deren Strukturformeln in Figur 5 dargestellt sind:
A1 = Ethyl-2,4-dihydroxy-3,6-dimethylbenzoat; Ethylatratat
A2 = Propyl-2,4-dihydroxy-3,6-dimethylbenzoat; Propylatratat
A3 = Butyl-2,4-dihydroxy-3,6-dimethylbenzoat; Butylatratat
A4 = 2-[(Phenylsulfonyl)amino]ethyl-2,4-dihydroxy-3,6-dimethylbenzoat*
A5 = (2*E*)-3,7-Dimethylocta-2,6-dien-1-yl-2,4-dihydroxy-3,5-dimethylbenzoat; Geranyl-2,4-dihydroxy-3,5-dimethylbenzoat; Geranylatratat*
A6 = Isopropyl-2,4-dihydroxy-3,5-dimethylbenzoat; Isopropylatratat
*(nicht erfindungsgemäß)

Darüber hinaus wurden auch die folgenden, kommerziell erhältlichen Verbindungen, welche eine strukturelle Ähnlichkeit zur Atrarsäure aufweisen, hinsichtlich ihrer antiandrogenen Wirkung untersucht:
R0 = Ethyl-2,4-dihydroxy-6-methylbenzoat*
R1 = Methyl-3,5-dibromo-2,4-dihydroxy-6-methylbenzoat* R2 = Methyl-2-hydroxy-3-methylbenzoat*
R3 = Methyl-2,4-dihydroxybenzoat*
R4 = Methyl-2,4-dihydroxy-3-methylbenzoat
R5 = Methyl-2,6-dihydroxy-3,5-dimethylbenzoat* R6 = 2,4-Dihydroxy-3,6-dimethylbenzoesäure*
X = 1-(2-Hydroxy-4,6-dimethoxyphenyl)-ethanon; Xanthoxylin*
*(nicht erfindungsgemäß)

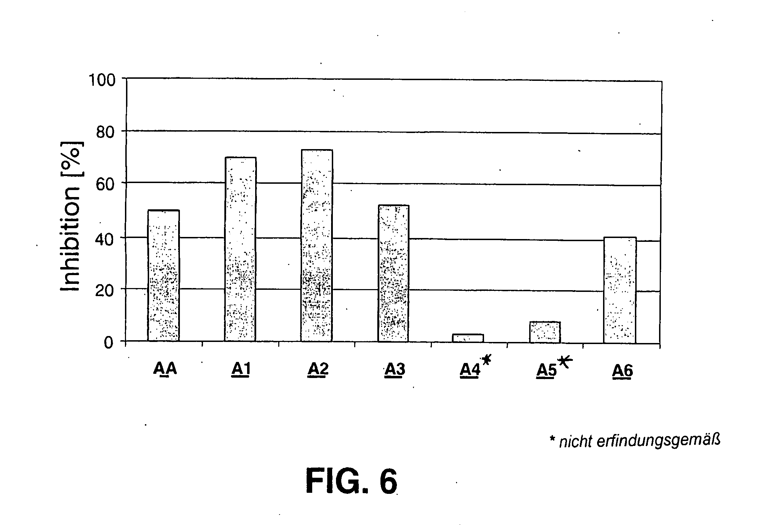

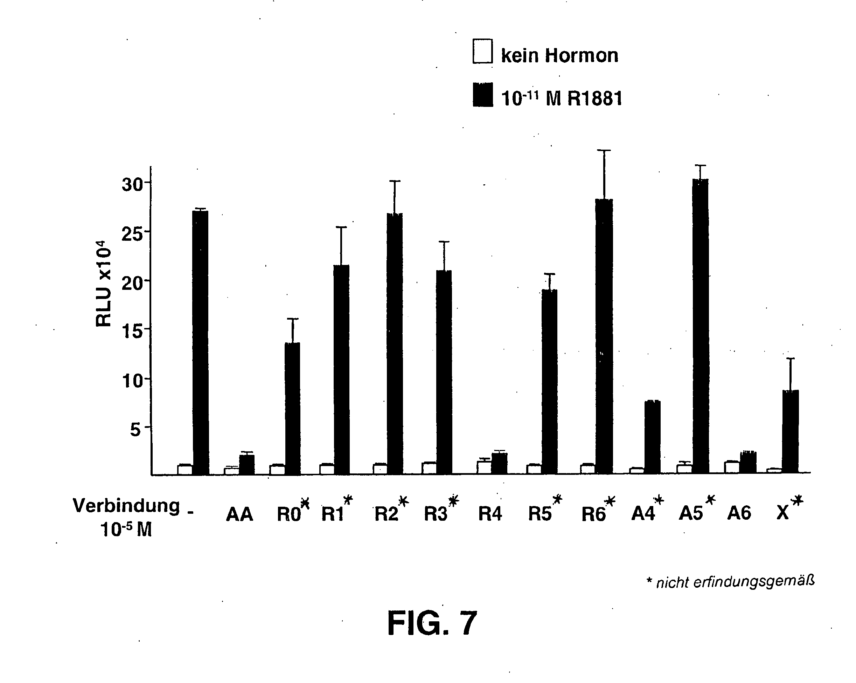

Die Verbindung A4 stellt eine Chimäre aus Atrarsäure und N-Butylbenzolsulfonamid dar, zeigt aber keine antiandrogene Wirkung, wie die Figuren 6 und 7 verdeutlichen. Das lässt vermuten, dass eine große Seitenkette als Ester bei einem Atrarsäure-Derivat nicht zu einem antiandrogen wirksamen Molekül führt. Diese Vermutung steht in Übereinstimmung damit, dass das Ersetzen der Isopropyl-Gruppe (Verbindung A6) durch die größere, hydrophobere Geranyl-Grupppe (Verbindung A5) nicht zu einem antiandrogen wirksamen Molekül führte.

Die Verbindungen R4 und A6 zeigten eine starke antiandrogene Wirkung, die bei einer Konzentration von 10 µM die Androgenrezeptor vermittelte Transaktivierung fast vollständig inhibierte (Figur 7). Selbst bei einer Konzentration von nur 1 µM waren beide Verbindungen, R4 und A6, noch in der Lage, die Androgenrezeptor-vermittelte Transaktivierung zu unterdrücken.

Die Ergebnisse der Luciferase-Assays legen nahe, dass die Methylgruppe an der *ortho*-Position für die antiandrogene Wirkung nicht erforderlich ist, wie ein Vergleich der Wirkungen von Atrarsäure und R4 zeigt, während eine Methyl-Gruppe an der meta-Position für die antiandrogene Wirkung essentiell ist (siehe Wirkung von R0). Damit in Übereinstimmung führt das Entfernen beider Methyl-Gruppen vom Benzol-Ring zu einer völligen Unterbindung der antiandrogenen Wirkung. Darüber hinaus können die Methyl-Gruppen nicht durch Bromatome ersetzt werden, ohne die antiandrogene Wirkung zu verlieren, wie die Aktivität von Verbindung R1 verdeutlicht. Auch die Verbindung R5, welche alle Substituenten am Benzolring aufweist, die auch bei der Atrarsäure vorhanden sind, jedoch an anderen Positionen, führt zu einem Verlust der antiandrogenen Aktivität. Auch die Methyl-Gruppe des Esters scheint essentiell für die antiandrogene Wirkung zu sein, da deren Entfernung die antiandrogene Wirkung unterbindet, wie die Wirkung von R6 aufzeigt.

Androgene sind für die normale Entwicklung, das normale Wachstum und die normalen sezernierenden Aktivitäten der Prostata unerläßlich. Im Unterschied dazu gelten Estrogene allgemein als Inhibitoren des Prostatawachstums. Allerdings dürfte eine derart allgemeine Bewertung von Estrogenen unzutreffend sein, denn es konnte gezeigt werden, dass eine Aktivierung des ERβ eine hemmende Wirkung auf das Wachstum von Prostatazellen hat. Darüber hinaus führte die Inaktivierung des ERβ zu einer Prostatahyperplasie bei Mäusen.

Im Rahmen der für die vorliegenden Erfindung gemachten Untersuchungen wurde auch gezeigt, dass Atrarsäure nicht nur eine antiandrogene Wirkung hat, sondern auch agonistisch auf den ERβ wirkt. Diese Erkenntnis legt nicht nur die Vermutung nahe, dass die hemmende Wirkung von Atrarsäure auf das Wachstum von Prostatakrebs-Zellen nicht allein auf ihrer antiandrogenen Wirkung sondern auch auf ihrem ERβ-Agonismus beruht. Vielmehr könnten auch Patienten mit anderen Erkrankungen, beispielsweise einer neurodegenerativen Erkrankung, von einer Behandlung mit Atrarsäure oder einem Atrarsäure-Derivat, das agonistisch auf den ERβ wirkt, profitieren.

Gegenstand der Erfindung ist daher die Verwendung eines 2,4-Dihydroxy-3-methylbenzoats der allgemeinen Formel 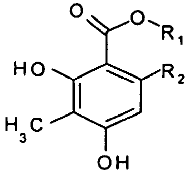 , wobei R₁ C₁- bis C₄-Alkyl bedeutet und R₂ Wasserstoff oder ein Methyl-, Ethyl- oder Propylrest ist, zur Herstellung eines Arzneimittels zur Behandlung des Prostatakarzinoms, insbesondere des gegen Androgen-Antagonisten therapieresistenten Prostatakarzinoms.

Atrarsäure kann aus biologischem Material mittels eines Verfahrens isoliert werden, das die folgenden Schritte umfasst:
a. Zerkleinern des biologischen Materials,
b. Extrahieren des biologischen Materials mit einem Lösungsmittel, das aus der Gruppe ausgewählt ist, die einwertige C₁- bis C₄-Alkohole und leichtflüchtige, (teil)-halogenierte C₁-Kohlenwasserstoffe umfasst;
c. Fraktionieren des Extrakts,
d. Isolierung von Atrarsäure aus den Atrarsäure enthaltenden Fraktionen.

Bei dem biologischen Material kann es sich um die Rinde des afrikanischen Pflaumenbaums *P. africana* oder um auf der Rinde von *P. africana* lebensfähige Flechten handeln. Vorzugsweise erfolgt die Extraktion als selektive Extraktion durch eine Reihe von aufeinander folgenden Lösungsmitteln mit zunehmender Polarität und die Fraktionierung des Extraktes mittels Gradientenextrographie, wobei die Polarität der Eluenten zunimmt. Besonders bevorzugt erfolgt die Isolierung der Atrarsäure aus den Atrarsäure-enthaltenden Fraktionen mittels präparativer HPLC.

Die Synthese von Atrarsäure-Derivaten (Atratate) kann mittels eines Verfahrens erfolgen, welches durch Rühren von Atrarsäure zusammen mit einer äquimolaren Menge an Alkalihydroxid oder Erdalkalihydroxid in einer Lösung eines primären aliphatischen Alkohols, wobei lediglich eine Umesterung eintritt, und anschließender Isolierung des Atrarsäure-Derivats aus dem Reaktionsgemisch, vorzugsweise mittels präparativer HPLC, gekennzeichnet ist.

Als Alkalihydroxid wird vorzugsweise Kaliumhydroxid verwendet und der primäre aliphatische Alkohol ist besonders bevorzugt aus der Gruppe ausgewählt, die Methanol, Ethanol, n-Propanol, iso-Propanol und Butanol umfasst.

### Beispiel 1: Extraktion des Pflanzenmaterials

Getrocknete Rinde des afrikanischen Pflaumenbaums *(P. africana*) wurde pulverisiert und 1,73 kg der pulverisierten Rinde wurden in 1 Liter n-Hexan mittels eines Ultra Turray mit Eis gekühlt homogenisiert. Das Pflanzenmaterial wurde in eine Säule (Merck Prepbar® 400 x 100 mm) gefüllt und nacheinander selektiv mit 25,0 Liter n-Hexan, 26,0 Liter Methylenchlorid, 25,0 Liter Methanol (MeOH) und 12,5 Liter Wasser bei Raumtemperatur extrahiert. Die Lösungsmittel der resultierenden Extrakte wurden unter Vakuum bei 40 °C verdampft. Es wurden 4,8 Gramm des selektiven Hexan-Extrakts, 11,03 Gramm des selektiven Methylenchlorid-Extrakts, 116,81 Gramm des selektiven Methanol-Extrakts und 7,0 Gramm des selektiven Wasser-Extrakts erhalten.

Zur Herstellung eines Ethanol-Extrakts wurde 300 Gramm Rindenmaterial von *P. africana* pulverisiert und dreimal mit je 5,0 Litern Ethanol (EOH) extrahiert. Nach Filtration des Extrakts durch Filterpapier mit einer Porengröße von 0,7 µm wurde das Lösungsmittel des gesamten Extrakts mittels eines Rotationsverdampfers bei 40 °C entfernt. Der resultierende Extrakt wies eine Trockenmasse von 16,02 Gramm auf.

### Beispiel 2: Fraktionierung des Methylenchlorid-Extrakts

Der selektive Methylenchlorid-Extrakt von *P. africana* wurde mit Hilfe von Silikagel (Machery - Nagel Si60, 15 - 25 µm) fraktioniert. Zu diesem Zweck wurde der Extrakt in 2000 ml CH₂Cl₂ gelöst und durch Filterpapier mit einer Porengröße von 0,7 µm (Schleicher & Schüll) filtriert. Fünfundzwanzig Gramm Silikagel (Merck Si60, 0,063 - 0;2 mm) wurden zu dem Extrakt gegeben und anschließend wurde das Lösungsmittel im Vakuum bei 40 °C verdampft. Das so beschichtete Silikagel wurde oben auf eine trocken gepackte Silikagel-Säule (Merck Prepbar® 400 X 100 mm) platziert und bei einer Flussrate von 120 ml·min⁻¹ mit einem linearen Gradienten von 0 min Hexan (100:0), 50 min Hexan (100:0), 350 min CH₂Cl₂ (100:0), 500 min CH₂Cl₂ (100:0), 700 min CH₂Cl₂-MeOH (80:20), 750 min MeOH (100:0), 800 min MeOH (100:0), 850 min H₂O (100:0), 885 min H₂O eluiert. Die Chromatographie ergab 35 Fraktionen, die zu einem Nachweis mit UV-Licht bei einer Wellenlänge von 245 nm führten (Tabelle 1).

**Tabelle 1: Fraktionierung des selektiven Methylenchlorid-Extrakts von P. africana**

| Fraktion | Min | Masse (mg) | Fraktion | Min | Masse (mg) |
|---|---|---|---|---|---|
| F1 | 0-148 | 3 | F19 | 615-630 | 799 |
| F2 | 149-184 | 52 | F20 | 631-638 | 292 |
| F3 | 185-204 | 33 | F21 | 639-659 | 1338 |
| F4 | 205-229 | 63 | F22 | 660-663 | 20 |
| F5 | 230-238 | 14 | F23 | 664-671 | 327 |
| F6 | 239-261 | 61 | F24 | 672-692 | 634 |
| F7 | 262-266 | 30 | F25 | 693-703 | 157 |
| F8 | 267-293 | 243 | F26 | 704-724 | 333 |
| F9 | 294-331 | 380 | F27 | 725-749 | 350 |
| F10 | 332-338 | 17 | F28 | 750-771 | 393 |
| F11 | 339-356 | 164 | F29 | 772-784 | 316 |
| F12 | 357-369 | 119 | F30 | 785-803 | 141 |
| F13 | 370-373 | 38 | F31 | 804-820 | 57 |
| F14 | 374-375 | 71 | F32 | 821-828 | 58 |
| F15 | 376-562 | 110 | F33 | 829-836 | 1 |
| F16 | 563-581 | 44 | F34 | 837-858 | 126 |
| F17 | 582-592 | 24 | F35 | 859-880 | 1 |
| F18 | 593-614 | 1537 | | | |

### Beispiel 3: Isolierung von Atrarsäure

Atrarsäure wurde aus Fraktion F8 durch präparative HPLC (250 x 21 mm, 100-5 C18 HD Machery - Nagel, 22 ml•min⁻¹, UV-Nachweis bei 220 nm, Gradient: 0 min ACN-H₂O (mit Zusatz von 0,1 % TFA) ((20:80), 40 min ACN-H₂O (80:20), 45 min ACN (Acetonitril) (100:0) isoliert. Atrarsäure wurde von Minute 23 bis Minute 25 gesammelt. Ihre Struktur wurde auf Grundlage der ¹H- und ¹³C- NMR, EI-MS, HR-EI-MS, IR und UV-Spektren aufgeklärt.

### Beispiel 4: Zellkultur und Luciferase-Assay

Nierenzellen von Affen, Linie CV1, die keinen endogenen Androgen-Rezeptor aufweisen, wurden in Dulbecco's modifiziertem Eagle's Medium (DMEM), ergänzt mit 10 % (v/v) fötalem Kälberserum, Penicillin (100 UI/ml) und Streptomycin (100 UI/ml) bei 37 °C und 5 % CO₂ kultiviert.

Für die Transfektionsexperimente wurden die Zellen in 6-Loch Zellkultur-Platten (Nunc, Roskilde, DK) in einer Dichte von 1,2 x 10⁵ zellen pro Loch ausgesät und in DMEM-Medium kultiviert, das mit 10 % (v/v) Dextran beschichteter Aktivkohle vermindertem Serum ergänzt war. Sechs Stunden nach Aussaat wurden die Zellen mittels der Ca₃(PO₄)₂-Methode transfiziert. Der Expressionsvektor für den menschlichen Androgen-Rezeptor (hAR)(0,2 pg) wurde mit 1 µg des Reporter-Plasmids MMTV-luc und 0,2 µg des Cytomegalovirus (CMV) getriebenen β-Galactosidase Expressionsvirus, als interne Kontrolle für die Transfektionseffizienz, cotransfiziert. Nach 14 h wurde das Medium entweder ohne (weiße Säulen in den Figuren 1 bis 3) oder mit Zugabe von Methyltrienolon (R1881, 3x10⁻¹⁰ M Endkonzentration; schwarze Säulen in den Figuren 1 bis 3) zusammen mit den angegebenen Extrakten (FIG. 1), Fraktionen des Methylenchloridextraktes (FIG. 2) oder isolierten Einzelverbindungen (FIG. 3) ersetzt. Nach weiteren 48 Stunden wurden die Zellen geerntet und auf Luciferase- und β-Glaktosidase-Aktivität hin untersucht.

Die Luciferase-Aktivität wurde durch Injektion von Luciferin und Messung der Lichtemission bei 562 nm bestimmt und als relative Lichteinheiten (RLU) angegeben, indem die Werte für β-Galaktosidase-Aktivität zur Normalisierung der Luciferase-Aktivität verwendet wurden. Sämtliche Transfektionsversuche wurden doppelt durchgeführt und zumindest zweimal wiederholt.

Zur Bestimmung der antiandrogenen Aktivität in verschiedenen Extrakten aus der Rinde von *P. africana* wurden die Extrakte in einer Konzentration von 300 µg/ml eingesetzt. Die Ergebnisse sind in FIG. 1 dargestellt.

Zur Bestimmung der antiandrogenen Aktivität in den Fraktionen des selektiven Methylenchlorid-Extrakts wurden zwei Mikroliter der jeweiligen Fraktion, was einer Endkonzentration von 30 µg/ml entspricht, verwendet. Bei den Fraktionen F6 bis F10 wurden zusätzlich Versuche mit 4 µl, entsprechend 60 µg/ml Endkonzentration, durchgeführt. Die aktiven Fraktionen F7 und F8 wurden für die weiteren Untersuchungen verwendet. Ein Teil der Ergebnisse ist in FIG. 2 dargestellt.
Die Inhibition der aus Fraktion F8 des selektiven Methylenchloridextraktes isolierten Substanzen wird in Figur 3 als Inhibition der Androgenwirkung in Prozent dargestellt. Die Substanzen wurden jeweils in einer Konzentration von 30 µg/ml eingesetzt.

### Beispiel 5: Inhibition des Wachstums von menschlichen Prostata-Karzinomzellen durch Atrarsäure

Menschliche Prostata-Karzinomzellen (Zelllinie LNCaP) wurden in RPMI-1640 Medium, das mit 10 % (v/v) fötalem Kälberserum, Penicillin (100 IU/ml), Streptomycin (100 IU/ml), 2 mM Glutamin und 1 mM Natriumpyruvat ergänzt wurde, gehalten.

Für die Wachstums-Untersuchungen wurden die LNCaP-Zellen mit einer Dichte von 5 x 10³ Zellen pro Loch in eine 24-Loch Zellkultur-Platte gesät und in RPMI-1640 Medium mit 5 % fötalem Kälberserum kultiviert. Am 2. Tag wurde das Kulturmedium gewechselt und zur Behandlung der Zellen Ethanol/DSMO (Kontrolle), Atrarsäure (1 µM und 10 µM) oder dem bekannten Antiandrogen Hydroxyflutamid (OH-F1) (0,1 µM) zugesetzt. Jeden zweiten Tag wurde das Medium mit den Zusätzen erneuert. An den angegebenen Tagen wurden die Zellen mit Trypsin behandelt und mit Hilfe einer Zählkammer gezählt. Die Ergebnisse sind in Figur 5 dargestellt.

### Beispiel 6: Synthese von Ethylatratat (A1)

Summenformel: C₁₁H₁₄O₄ (MG = 210,09)
IUPAC: Ethyl-2,4-dihydroxy-3,6-dimethylbenzoat
Erscheinungsform: weißes Pulver

### Synthese:

392 mg Atrarsäure (2 mmol) wurden mit 118 mg Kaliumhydroxid (2,1 mmol) in 10 ml Ethanol über Nacht gerührt und dann neutralisiert. Das Lösungsmittel wurde am Rotationsverdampfer entfernt und der Rückstand im Lösungsmittelgemisch der chromatographischen Anfangsbedingungen gelöst. Anschließend erfolgte die Isolierung des Reaktionsproduktes per präparativer HPLC gemäß Methode B4:

| | | | | |
|---|---|---|---|---|
| B4 | Macherey - Nagel | A: Acetonitril / 0,1% TFA | 22,0 ml/min | PDA: λ = 220 nm |
| | Nucleosil® 100-5-C-18HD, 5 µm, 250 x 21 mm | B: Wasser / 0,1% TFA | | |
| | | Isokratisch: 50% A, 50% B | | |

Retentionszeit 13 Min.
Ausbeute: 42 mg (10%)
Schmelzpunkt (°C): 127
UV (MeOH) λₘₐₓ nm: 217, 265, 308
IR (KBr) νₘₐₓ cm⁻¹: 3450, 3100, 1620, 1310, 1280, 800
¹H-NMR (500 MHz, CDCl₃), δ (ppm):
12,05 (1H, s, C-2-OH) 6,14 (1H, s, C-5-H) 5,02 (1H, s, C-4-OH) 4, 32 (2H, q, ³J = 7,0 Hz, C-1'-H) 2,41 (3H, *s*, C-6-Me) 2,03 (3H, *s,* C-3-Me) 1,34 (3H, *t,* ³J = 7,0 Hz, C-2'-H) ¹³C-NMR (125 MHz, CDCl₃), δ (ppm):

| | | |
|---|---|---|
| 172,1 (C-7) | 108,5 (C-3) | 7,6 (C-3-Me) |
| 163,2 (C-2) | 105,4 (C-1) | |
| 157,9 (C-4) | 61,2 (C-1') | |
| 140,2 (C-6) | 24,6 (C-6-Me) | |
| 110,5 (C-5) | 14,2 (C-2') | |

EI-MS (70 eV): *m*/*z* (rel. int.):
210 [M]⁺ (50), 164 (100), 136 (60)

Massenfeinbestimmung (HR-EI-MS):
Berechnet: 210,0892 für [M⁺]
Gefunden: 210,0889

### Beispiel 7: Synthese von Propylatratat (A2)

Summenformel: C₁₂H₁₆O₄ (MG = 224,10)
IUPAC: Propyl-2,4-dihydroxy-3,6-dimethylbenzoat
Erscheinungsform: weißes Pulver

### Synthese:

392 mg Atrarsäure (2) (2 mmol) wurden mit 118 mg Kaliumhydroxid (2,1 mmol) in 10 ml Propanol über Nacht gerührt und dann neutralisiert. Das Lösungsmittel wurde am Rotationsverdampfer entfernt und der Rückstand im Lösungsmittelgemisch der chromatographischen Anfangsbedingungen gelöst. Anschließend erfolgte die Isolierung des Reaktionsproduktes per präparativer HPLC gemäß Methode B5:

| | | | | |
|---|---|---|---|---|
| B5 | Macherey - Nagel | A: Methanol | 22,0 ml/min | PDA: |
| | | B: Wasser / 0,1% TFA | | λ = 220 nm |
| | Nucleosil® 100-5-C-18HD, 5 µm, 250 x 21 mm | Isokratisch: 72% A, 28% B | | |

Retentionszeit 10 Min.
Ausbeute: 31 mg (7%)
Schmelzpunkt (°C): 134
UV (MeOH) λₘₐₓ nm: 217, 262, 307
IR (KBr) νₘₐₓ cm⁻¹: 3450, 3000, 1650, 1310, 1200, 800
¹H-NMR (500 MHz, CDCl₃), δ (ppm) :
12,09 (1H, s, C-2-OH)
6,14 (1H, s, C-5-H)
5,02 (1H, s, C-4-OH)
4,23 (2H, t, ³J = 6,7 Hz, C-1'-H)
2,41 (3H, *s,* C-6-Me)
2,04 (3H, *s,* C-3-Me)
1,73 (2H, *m,* ³J = 7,0 Hz, C-2'-H)
0,97 (3H, *t*, ³J = 7,2 Hz, C-3'-H)
¹³C-NMR (125 MHz, CDCl₃), δ (ppm) :

| | | |
|---|---|---|
| 173,3 (C-7) | 108,5 (C-3) | 10,8 (C-3') |
| 163,2 (C-2) | 105,4 (C-1) | 7,6 (C-3-Me) |
| 157,9 (C-4) | | 67,0 (C-1') |
| 140,1 (C-6) | | 24,2 (C-6-Me) |
| 110,5 (C-5) | | 22,0 (C-2') |

EI-MS (70 eV): *m*/*z* (rel. int.):
224 [M]⁺ (31), 164 (100), 136 (44)
Massenfeinbestimmung (HR-EI-MS):
Berechnet: 224,1049 für [M⁺]
Gefunden: 224,1051

### Beispiel 8: Synthese von Butylatratat (A3)

Summenformel: C₁₃H₁₈O₄ (MG = 238, 12 )
IUPAC: Butyl-2,4-dihydroxy-3,6-dimethylbenzoat
Erscheinungsform: weißes Pulver

### Synthese:

392 mg Atrarsäure (2) (2 mmol) wurden mit 118 mg Kaliumhydroxid (2,1 mmol) in 10 ml Butanol über Nacht gerührt und dann neutralisiert. Das Lösungsmittel wurde am Rotationsverdampfer entfernt und der Rückstand im Lösungsmittelgemisch der chromatographischen Anfangsbedingungen gelöst. Anschließend erfolgte die Isolierung des Reaktionsproduktes per präparativer HPLC gemäß Methode B7:

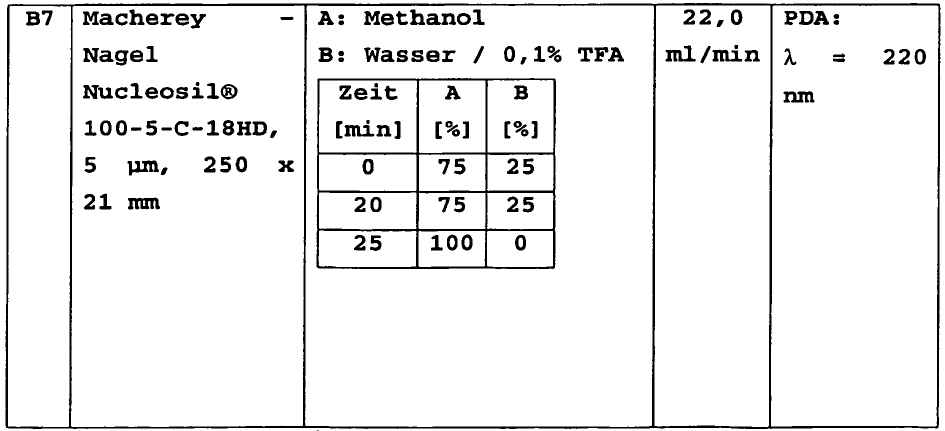

Retentionszeit 13 Min.
Ausbeute: 51 mg (11%)
Schmelzpunkt (°C): 117
UV (MeOH) λₘₐₓ nm: 217, 265, 308
IR (KBr) νₘₐₓ cm⁻¹: 3440, 3000, 1700, 1310, 1200, 800
¹H-NMR (500 MHz, CDCl₃), δ (ppm):
12,09 (1H, *s,* C-2-OH)
6,20 (1H, *s,* C-5-H)
4,97 (1H, *s,* C-4-OH)
4,32 (2H, *t,* ³J = 7,0 Hz, C-1'-H)
2,41 (3H, *s,* C-6-Me)
2,04 (3H, *s,* C-3-Me)
1,69 (2H, *m*, ³J = 7,3 Hz, C-2'-H)
1,42 (2H, *m,* ³J = 7,3 Hz, C-3'-H)
0,90 (3H, *t,* ³J = 7,3 Hz, C-4'-H)

¹³C-NMR (125 MHz, CDCl₃), δ (ppm) :

| | | |
|---|---|---|
| 167,9 (C-7) | 108,6 (C-3) | 19,3 (C-3') |
| 163,1 (C-2) | 105,5 (C-1) | 13,5 (C-4') |
| 157,8 (C-4) | 66,3 (C-1') | 7,5 (C-3-Me) |
| 140,1 (C-6) | 24,1 (C-6-Me) | |
| 110,5 (C-5) | 30,5 (C-2') | |

EI-MS (70 eV): m/z (rel. int.):
238 [M]⁺ (31), 164 (100), 136 (30)

Massenfeinbestimmung (HR-EI-MS):
Berechnet: 238,1226 für [M⁺]
Gefunden: 238,1226

### Beispiel 9: Synthese eines Hybrids aus Atrarsäure und N-Butylbenzolsulfonamid (A4); (nicht erfindungsgemäß)

Summenformel: C₁₇H₁₉O₆NS (MG = 365,09)
IUPAC: 2-[(Phenylsulfonyl)amino]ethyl 2,4-dihydroxy-3,6-dimethylbenzoat
Erscheinungsform: gelbliches Pulver

### Synthese:

1,822 g 2,4-Dihydroxy-3,6-dimethylbenzoesäure (0,01 mol) und 3,522 g *N-*(2-Hydroxyethyl)benzolsulfonamid wurden in 30 ml *ortho*-Toluol unter Zugabe von 0,05 g Schwefelsäure konz. 5 Stunden unter Rückfluss behandelt. Nach Neutralisieren wurde das Lösungsmittel am Rotationsverdampfer entfernt und der Rückstand in dem Lösungsmittelgemisch der chromatographischen Anfangsbedingungen gelöst. Anschließend erfolgte die Isolierung des Reaktionsproduktes per präparativer HPLC gemäß Methode B9:

| | | | | |
|---|---|---|---|---|
| B9 | Macherey - Nagel | A: Methanol | 22,0 ml/min | PDA: λ = 220 nm |
| | | B: Wasser / 0,1% TFA | | |
| | Nucleosil® 100-5-C-18HD, 5 µm, 250 x 21 mm | Isokratisch: 60% A, 40% B | | |

Retentionszeit 14 Min.
Ausbeute: 30 mg (4%)
Schmelzpunkt (°C): 151
UV (MeOH) λₘₐₓ nm: 220, 270, 305
IR (KBr) νₘₐₓ cm⁻¹: 3450, 3310, 2930, 1640, 1310, 1280, 1160, 1100
¹H-NMR (500 MHz, CDCl₃), δ (ppm):
11,69 (1H, s, C-2-OH)
7,79 (2H, d, ³J = 7,0 Hz, C-2''-H und C-6''-H)
7,48 (1H, t, ³J = 7,0 Hz, C-4'' -H)
7,41 (2H, t, ³J = 7,0 Hz, C-3''-H und C-5''-H)
6,13 (1H, s, C-5-H)
5,05 (1H, s, C-4-OH)
4,68 (1H, s, N-H)
4,31 (2H, t, ³J = 5,5 Hz, C-1'-H)
3,32 (2H, t, ³J = 5,5 Hz, C-2'-H)
2,31 (3H, s, C-6-Me)
2,03 (3H, s, C-3-Me)

¹³C-NMR (125 MHz, CDCl₃), δ (ppm):

| | | |
|---|---|---|
| 167,0 (C-7) | 133,6 (C-4") | 104,2 (C-1) |
| 163,0 (C-2) | 130,2 (C-3" und C-5'') | 64,5 (C-1') |
| 153,1 (C-4) | 127,8 (C-2" und C-6'') | 43,0 (C-2') |
| 142,0 (C-6) | 111,6 (C-5) | 24,6 (C-6-Me) |
| 141,4 (C-1'') | 109,9 (C-3) | 7,5 (C-3-Me) |

EI-MS (70 eV): m/z (rel. int.):
365 [M]⁺ (23), 170 (26), 164 (100), 141 (26), 136 (24), 77 (27)

Massenfeinbestimmung (HR-EI-MS):
Berechnet: 365,0933 für [M⁺]
Gefunden: 365,0933

### Beispiel 10: Synthese von Geranylatratat (A5) (nicht erfindungsgemäß)

Summenformel: C₁₉H₂₆O₄ (MG = 318,18)
IUPAC: (2E)-3,7-Dimethylocta-2,6-dien-1-yl 2,4-dihydroxy-3,6-dimethylbenzoat
Erscheinungsform: gelbliches Pulver

### Synthese:

392 mg Atrarsäure (2) (2 mmol) wurden mit 118 mg Kaliumhydroxid (2,1 mmol) in 10 ml Geraniol über Nacht gerührt und dann neutralisiert. Das Lösungsmittel wurde am Rotationsverdampfer entfernt und der Rückstand in dem Lösungsmittelgemisch der chromatographischen Anfangsbedingungen gelöst. Anschließend erfolgte die Isolierung des Reaktionsproduktes per präparativer HPLC gemäß Methode B8:

| | | | | |
|---|---|---|---|---|
| B8 | Macherey - Nagel | A: Acetonitril / 0,1% TFA | 22,0 ml/min | PDA: λ = 220 nm |
| | Nucleosil® | B: Wasser / 0,1% TFA | | |
| | 100-5-C-18HD, 5 µm, 250 x 21 mm | Isokratisch: 50% A, 50% B | | |

Retentionszeit 27 Min.
Ausbeute: 44 mg (7%)
UV (ACN) λₘₐₓ nm: 220, 270, 310
IR (KBr) vₘₐₓ cm⁻¹: 3410, 2930, 1640, 1440, 1270
¹H-NMR (500 MHz, MeOH-*d4*), δ (ppm):
6,20 (1H, *s*, C-5-H)
2,45 (3H, *s*, C-6-Me)
5,34 (1H, *t,* ³J = 6,7 Hz, C-2'-H)
5,10 (1H, *t,* ³J = 6,7 Hz, C-6'-H)
4,07 (2H, *d,* ³J = 6,5 Hz, C-1'-H)
2,10 (2H, *q*, ³J = 8,3 Hz, C-5'-H)
2,01 (2H, *t,* ³J = 8,3 Hz, C-4'-H)
1,99 (3H, *s*, C-3 Me)
1,65 (3H, *s*, C-3'-Me)
1,64 (3H, *s*, C-7'-Me)
1,59 (3H, *s,* C-8')

¹³C-NMR (125 MHz, MeOH-d4), δ (ppm):

| | | | |
|---|---|---|---|
| 175,6 (C-7) | 132,4 (C-7') | 104,9 (C-1) | 24,3 (C-6-Me) |
| 164,8 (C-2) | 125,1 (C-2') | 59,4 (C-1') | 17,7 (C-5') |
| 161,3 (C-4) | 124,9 (C-6') | 40,7 (C-4') | 16,2 (C-3'-Me) |
| 141,5 (C-3') | 111,3 (C-5) | 27,5 (C-8') | 7,9 (C-3-Me) |
| 139,4 (C-6) | 109,7 (C-3) | 25,8 (C-7'-Me) | |

EI-MS (70 eV): *m*/*z* (rel. int.):
318 [M]⁺ (24), 164 (100), 136 (38)

Massenfeinbestimmung (HR-EI-MS):
Berechnet: 318,1834 für [M⁺]
Gefunden: 318,1829

### Beispiel 11: Synthese von Isopropylatratat (A6)

Summenformel: C₁₂H₁₆O₄ (MG = 224,10)
IUPAC: Isopropyl-2,4-dihydroxy-3,6-dimethylbenzoat
Erscheinungsform: weißes Pulver

### Synthese:

392 mg Atrarsäure (2) (2 mmol) wurden mit 118 mg Kaliumhydroxid (2,1 mmol) in 10 ml iso-Propanol über Nacht gerührt und dann neutralisiert. Das Lösungsmittel wurde am Rotationsverdampfer entfernt und der Rückstand in dem Lösungsmittelgemisch der chromatographischen Anfangsbedingungen gelöst. Anschließend erfolgte die Isolierung des Reaktionsproduktes per präparativer HPLC gemäß Methode B6:

| | | | | |
|---|---|---|---|---|
| B6 | Macherey - Nagel | A: Methanol | 22,0 ml/min | PDA: λ = 220 nm |
| | | B: Wasser / 0,1% TFA | | |
| | Nucleosil® 100-5-C-18HD, 5 µm, 250 x 21 mm | Isokratisch: 70% A, 30% B | | |

Retentionszeit 12 Min.
Ausbeute: 38 mg (8%)
Schmelzpunkt (°C): 90
UV (MeOH) λₘₐₓ nm: 217, 265, 300
IR (KBr) νₘₐₓ cm⁻¹: 3400, 3000, 1650, 1310, 1200, 800
¹H-NMR (500 MHz, CDCl₃), δ (ppm) :
12,11 (1H, s, C-2-OH)
6,13 (1H, s, C-5-H)
5,22 (1H, m, ³J = 6,3 Hz, C-1'-H)
2,40 (3H, s, C-6-Me)
2,03 (3H, *s,* C-3-Me)
1,31 (6H, *d,* ³J = 6,2 Hz, C-1'-Me)

¹³C-NMR (125 MHz, CDCl₃), δ (ppm):

| | | |
|---|---|---|
| 171,6 (C-7) | 108,5 (C-3) | 7,6 (C-3-Me) |
| 163,2 (C-2) | 105,7 (C-1) | |
| 157,8 (C-4) | 69,2 (C-1') | |
| 140,1 (C-6) | 24,3 (C-6-Me) | |
| 110,4 (C-5) | 22,0 (C-1'-Me) | |

EI-MS (70 eV): *m*/*z* (rel. int.):
224 [M]⁺ (35), 164 (100), 136 (39)

Massenfeinbestimmung (HR-EI-MS):
Berechnet: 224,1049 für [M⁺]
Gefunden: 224,1051

### Beispiel 12: Atrarsäure als Agonist für Estrogenrezeptoren

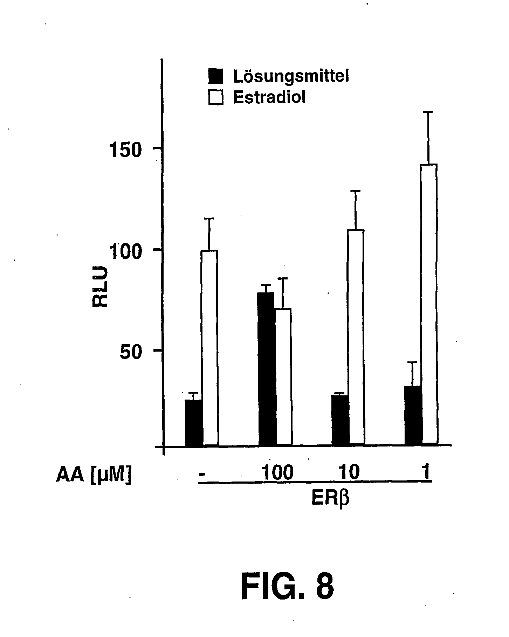

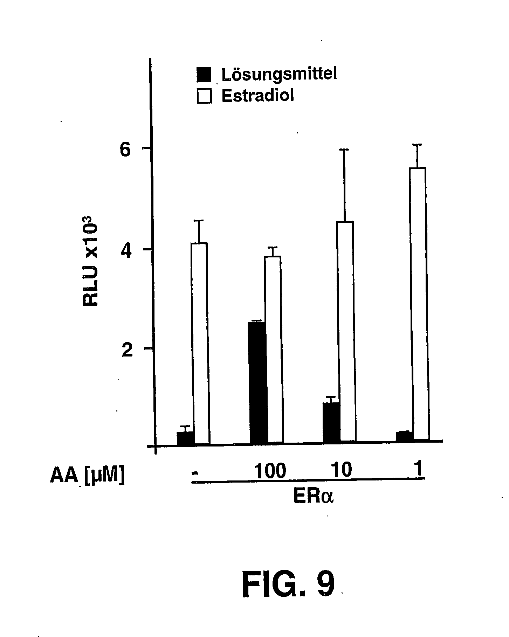

CV1-Zellen, die keine signifikanten Mengen eines funktionellen Estrogenrezeptors aufweisen, wurden für funktionelle Untersuchungen mit einem Expressionsplasmid, das das Gen für Liciferase als Reportergen kodiert (p2ERE-TATA-luc), und einem Expressionsplasmid co-transfiziert, das den menschlichen ERα oder den menschlichen ERβ kodiert. Für die transienten Transfektionsexperimente mit den Estrogenrezeptoren wurde Phenolrot-freies DMEM-Medium (Invitrogen) mit 10 % (v/v) Serum, das zuvor mittels Dextran-beschichteter Kohle gereinigt worden war, 1% (v/v) Glutamin, 1% (v/v) Natriumpyruvat und 1% (v/v) Penicillin-Streptomycin ergänzt. Die Zellen wurden nach sieben Tagen mit einer Dichte von 1,5x10⁵ Zellen pro Loch in 6-LochPlatten (Nunc, Roskilde, DK) ausgesät. Nach 24 Stunden wurden die Zellen gemäß der Calciumphosphat-Methode mit 2 µg des Reportergen-kodierenden Expressionsplasmids, 0,2 pg des ERα- oder des ERβ-kodierenden Expressionsplasmids und, der Normalisierung wegen, 0,2 µg der vom Cytomegalovirus stammenden β-Galaktosidase transfiziert. 14 Stunden später wurde das Kulturmedium gegen frisches Medium mit oder ohne Estradiol ausgetauscht, dem jedenfalls eine entsprechende Menge Atrarsäure zugesetzt war. Nach weiteren 48 Stunden wurden die Zellen geerntet und auf Luciferase- und β-Galaktosidase-Aktivität untersucht. Sämtliche Transfektionsversuche wurden in Duplikat durchgeführt und mindestens zweimal wiederholt. Die Ergebnisse sind in den Figuren 8 und 9 graphisch dargestellt. Die Versuchsergebnisse zeigen, dass die Aktivität der beiden Estrogenrezeptoren durch Atrarsäure beeinflusst wird. Überraschenderweise wurde die Hormonvermittelte Transaktivierung der beiden Estrogenrezeptoren durch Atrarsäure nicht beeinträchtigt. Bei Abwesenheit von Estradiol wurde jedoch die auf Estrogene ansprechende Expression des Reportergen durch Gegenwart vom 10 µM oder 100 µM in dosisabhängiger Weise aktiviert, wie die gemessene Luciferase-Aktivität erkennen lässt. Atrarsäure ist also bei höheren Konzentrationen ein Agonist beider Estrogenrezeptoren.

## Patentansprüche

1. Verwendung eines 2,4-Dihydroxy-3-methylbenzoats der allgemeinen Formel (I) 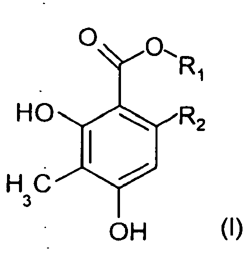
wobei R₁ C₁- bis C₄-Alkyl bedeutet und R₂ Wasserstoff oder ein Methyl-, Ethyl- oder Propylrest ist,
zur Herstellung eines Arzneimittels zur Behandlung des Prostatakarzinoms.

2. Verwendung gemäß Anspruch 1, **dadurch gekennzeichnet, dass** R₂ in der Formel (I)Wasserstoff oder eine Methylgruppe darstellt.

3. Verwendung gemäß einem der Ansprüche 1 bis 2, **dadurch gekennzeichnet, dass** das Prostatakarzinom therapieresistent gegen Androgen-Antagonisten ist. 3

4. Verwendung gemäß Anspruch 3, **dadurch gekennzeichnet, dass** der Androgen-Antagonist aus der Bicalutamid, Flutamid, Hydroxyflutamid, Nilutamid und Cyproteronacetat umfassenden Gruppe ausgewählt ist.

## Claims

1. Use of a 2,4-dihydroxy-3-methylbenzoate of the general formula (I) 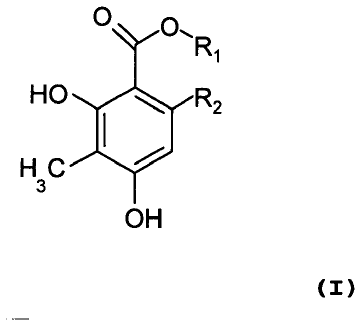
wherein R₁ represents a C₁ to C₄ alkyl, and R₂ is hydrogen or a methyl, ethyl or propyl residue,
for the manufacture of a medicament for the treatment of prostate carcinoma.

2. Use according to claim 1, **characterised in that** R₂ in formula (I) is hydrogen or a methyl group.

3. Use according to any one of claims 1 to 2, **characterised in that** the said prostate carcinoma is resistant to treatment with androgen antagonists

4. Use according to claim 3, **characterized in that** the androgen antagonist is selected from the group comprising bicalutamide, flutamide, hydroxyflutamide, nilutamide and cyproterone acetate.

## Revendications

1. Utilisation d'un benzoate de 2,4-dihydroxy-3-méthyle répondant à la formule générale (I) 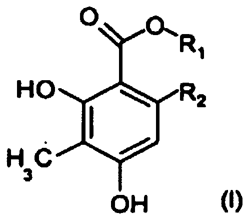
dans laquelle R₁ représente un groupe alkyle en C₁-C₄ et R₂ représente un atome d'hydrogène ou un radical méthyle, un radical éthyle ou un radical propyle,
pour la préparation d'un médicament destiné à traiter le carcinome de la prostate.

2. Utilisation selon la revendication 1, **caractérisée en ce que** R₂ dans la formule (I)représente un atome d'hydrogène ou un groupe méthyle.

3. Utilisation selon l'une quelconque des revendications 1 à 2, **caractérisée en ce que** le carcinome de la prostate résiste à une thérapie dirigée contre les antagonistes androgéniques.

4. Utilisation selon la revendication 3, **caractérisée en ce que** l'antagoniste androgénique est choisi parmi le groupe comprenant le bicalutamide, le flutamide, l'hydroxyflutamide, le nilutamide et l'acétate de cyprotérone.
